(19) Europäisches Patentamt
European Patent Office
Office européen des brevets

(11) Numéro de publication : **0 097 079**
**B1**

(12) # FASCICULE DE BREVET EUROPÉEN

(45) Date de publication du fascicule du brevet : **01.10.86**

(21) Numéro de dépôt : **83401110.8**

(22) Date de dépôt : **01.06.83**

(51) Int. Cl.[4] : **C 07 D495/04**// A61K31/435 ,(C07D495/04, 333:00, 221:00)

(54) **Nouveau dérivé de thiéno-pyridinone, son procédé de préparation et son utilisation thérapeutique.**

(30) Priorité : **16.06.82 FR 8210926**

(43) Date de publication de la demande : **28.12.83 Bulletin 83/52**

(45) Mention de la délivrance du brevet : **01.10.86 Bulletin 86/40**

(84) Etats contractants désignés : **AT BE CH DE FR GB IT LI LU NL SE**

(56) Documents cités :
**FR-A- 2 495 156**
**P.A.S. SMITH: "The chemistry of open-chain organic nitrogen compounds", vol. II, "Derivatives of oxidized nitrogen:hydrazines to nitrates", 1966, pages 39,64, Benjamin, New York, USA**
**Le dossier contient des informations techniques présentées postérieurement au dépôt de la demande et ne figurant pas dans le présent fascicule.**

(73) Titulaire : **SANOFI, Société dite: 40, Avenue George V F-75008 Paris (FR)**

(72) Inventeur : **Frehel, Daniel Résidence Grand Ramier Bat.C. Appt. 52-7 Avenue Pr. C.Soula F-31400 Toulouse (FR)**
Inventeur : **Maffrand, Jean-Pierre 5 Rue du Corps Franc Pommiès F-31120 Portet/Garonne (FR)**
Inventeur : **Vallee, Eric 253 Chemin du Ramelet Moundi F-31170 Tournefeuille (FR)**

(74) Mandataire : **Polus, Camille et al c/o Cabinet Lavoix 2, Place d'Estienne d'Orves F-75441 Paris Cedex 09 (FR)**

**EP 0 097 079 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

. La présente invention est relative à un nouveau dérivé de tétrahydro-5,6,7,7a 4H-thiéno (3,2-c) pyridinone-2, à son procédé de préparation et aux médicaments le contenant.

Le composé selon l'invention répond à la formule I

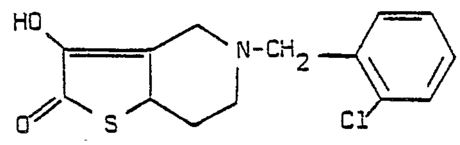

(I)

ainsi que ses sels d'addition avec les acides minéraux ou organiques pharmaceutiquement acceptables.

Ce composé comportant un carbone asymétrique peut exister sous la forme de deux énantiomères. L'invention concerne aussi bien chacun des énantiomères que leur mélange.

L'invention a également pour objet un procédé de préparation du composé de l'invention caractérisé en ce que

a) on soumet à une nitrosation le composé de formule II

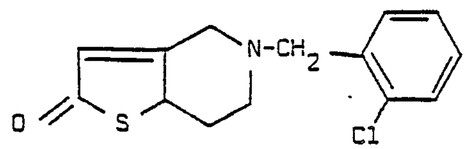

(II)

b) le céto-oxime ainsi obtenu de formule III

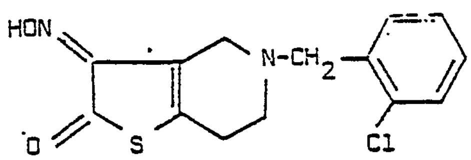

(III)

est ensuite hydrogéné

c) Le composé hydrogéné ainsi obtenu, de formule IV

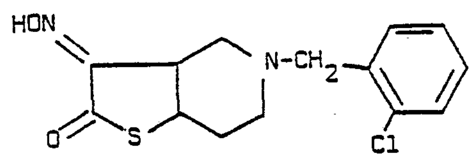

(IV)

est ensuite soumis à un traitement acide pour obtenir le dérivé de formule I.

Le composé de formule II, point de départ de la préparation du composé de l'invention est décrit ainsi que son procédé de préparation dans la demande de brevet n° 80 25 274 déposé par la Demanderesse.

L'opération de nitrosation permettant d'obtenir le composé de formule III c'est-à-dire la (chloro-2 benzyl)-5 (hydroxyimino)-3 tétrahydro-4,5,6,7 3H-thiéno (3,2-c) pyridinone-2 s'effectue en milieu acide acétique glacial sous atmosphère inerte en faisant réagir le composé de formule II avec le nitrite de sodium.

La phase d'hydrogénation a lieu en présence d'acide perchlorique et d'un catalyseur, de préférence du palladium sur charbon en solution dans un alcool tel que le méthanol, l'éthanol, l'isopropanol, ou un solvant hydroalcoolique, à une température variant entre 10 °C et 50 °C.

Le traitement acide est effectué sur le composé de formule IV en solution dans un mélange constitué d'un alcool tel que le méthanol, l'éthanol, l'isopropanol et d'un acide tel que l'acide chlorhydrique ou bromhydrique à des concentrations comprises entre N et 3N. L'opération s'effectue à reflux, sous atmosphère inerte et pendant une durée comprise entre 3 heures et 6 heures.

Les exemples non limitatifs suivants illustrent l'invention :

a) (Chloro-2 benzyl)-5 (hydroxyimino)-3 tétrahydro-4,5,6,7 3H-thiéno (3,2-c) pyridinone-2

On met en suspension 25 g (0,006 8 mole) d'oxalate de (chloro-2 benzyl)-5 tétrahydro-5,6,7,7a 4H-thiéno (3,2-c) pyridinone-2 dans 125 ml d'acide acétique glacial, sous atmosphère inerte et après refroidissement du milieu réactionnel entre 0 °C et 5 °C, on ajoute, goutte à goutte, 5 g (0,072 mole) de nitrite de sodium dissous dans 30 ml d'eau. On abandonne à température ambiante pendant 7 heures. Les

cristaux sont filtrés, lavés à l'éther et séchés. Le produit est recristallisé dans l'eau. Cristaux jaunes, F = 222 °C, rendement = 70 %.

b) (Chloro-2 benzyl)-5 (hydroxyimino)-3 hexahydro-3a,4,5,6,7,7a 3H-thiéno (3,2-c) pyridinone-2

On dissout 15 g (0,037 6 mole) du composé de formule III précédemment obtenu dans un mélange de 900 ml de méthanol et de 8 ml d'acide perchlorique à 70 %. On ajoute 4 g de palladium sur charbon à 10 % en suspension dans 100 ml d'éthanol, au milieu réactionnel. On soumet le mélange réactionnel à l'hydrogénation dans un réacteur à pression ordinaire (1 atm.) et l'abandonne pendant 6 heures à température ambiante. Après filtration du catalyseur, on évapore à sec le milieu réactionnel.

Le résidu dissous dans l'eau est neutralisé par une solution aqueuse saturée de bicarbonate de sodium. On extrait la phase aqueuse avec du dichlorométhane, sèche la phase organique sur sulfate de sodium sec et évapore à sec. Le résidu huileux est purifié par chromatographie sur une colonne de silice (élution : toluène-acétate d'éthyle 1/1).

La purification finale s'effectue par l'intermédiaire de l'oxalate. Cristaux beiges, F = 200 °C, rendement : 65 %.

c) (Chloro-2 benzyl)-5 hydroxy-3 tétrahydro-5,6,7,7a 4H-thiéno (3,2-c) pyridinone-2

On dissout 10,4 g (0,033 4 mole) du composé de formule IV obtenu à l'étape précédente dans un mélange de 250 ml d'alcool isopropylique et de 45 ml d'acide chlorhydrique 2N. On porte au reflux sous atmosphère inerte pendant 4 heures. On évapore à sec et reprend le résidu par une solution aqueuse saturée de bicarbonate de sodium. On extrait la phase aqueuse avec le dichlorométhane. La phase organique est séchée sur du sulfate de sodium sec et évaporée à sec. Le résidu huileux obtenu est purifié par filtration sur un lit de silice (élution : toluène-acétate d'éthyle 1/1). La base obtenue avec un rendement de 81 % se présente sous forme de cristaux beiges, F = 121 °C.

Différents sels ont été préparés selon les méthodes classiques : hémioxalate : cristaux crème, F = 170 °C (recristallisation dans un mélange d'alcool isopropylique et d'acetonitrile), nitrate : cristaux roses, F = 181 °C (recristallisation dans un mélange d'isopropanol et de méthanol), chlorhydrate : cristaux beiges, F = 139 °C (recristallisation dans l'isopropanol).

L'invention a encore pour objet un médicament ayant en particulier des propriétés anti-agrégante plaquettaire et anti-thrombotique, caractérisé en ce qu'il contient, à titre de principe actif, le composé de formule I, ou un sel d'addition avec un acide minéral ou organique pharmaceutiquement acceptable.

L'étude toxicologique qui a porté sur les toxicités aiguë, chronique, subchronique et retardée, a mis en évidence la bonne tolérance et la faible toxicité du dérivé de l'invention.

Les essais effectués sur les souris, rats et lapins ont montré que l'administration, aussi bien orale qu'intra-péritonéale, du composé de l'invention n'avait provoqué tout au long des différents essais pratiqués, aucune réaction locale ou générale, aucune perturbation dans les contrôles biologiques régulièrement effectués, aucune lésion macroscopique ou microscopique.

L'étude pharmacologique a mis en évidence des actions inhibitrices de l'agrégation plaquettaire et anti-thrombotique.

1°) Action inhibitrice de l'agrégation plaquettaire

Cette expérimentation est effectuée sur le rat qui a reçu, pendant 3 jours, par la voie orale, 100 mg du composé de l'invention aux moments — 48 h, — 24 h et — 2 h. Au moment 0 h, on prélève 4 ml de sang selon la technique de Renaud à la veine jugulaire de l'animal anesthésié. C'est ce sang citraté qui est utilisé dans les mesures d'agrégation.

a) mesure de l'agrégation plaquettaire à l'A.D.P.

2 ml de sang citraté sont rapidement versés dans un petit becher placé sur un agitateur magnétique et pourvu d'une barre aimantée. Après quelques secondes d'agitation, on introduit dans le becher 0,4 ml d'une solution contenant 0,66 μg d'adénosine-diphosphate (A.D.P.) par ml. Après 90 secondes d'agitation, on procède à deux prélèvements de 0,5 ml de sang :

— le premier est mélangé avec 0,5 ml d'une solution EDTA-Formol,

— le deuxième est mélangé avec 0,5 ml d'une solution EDTA seulement.

L'addition d'EDTA-formol a pour but de stabiliser le sang et donc de fixer l'agrégation tandis que l'EDTA provoque au contraire la désagrégation de tous les amas plaquettaires.

Après un repos de 10 minutes et une centrifugation des 2 mélanges à vitesse lente pendant 5 minutes, afin de séparer les globules rouges, le plasma riche en plaquettes (PRP) surnageant est prélevé, dilué et numéré en plaquettes.

L'intensité de l'agrégation est déterminée par le rapport

$$\frac{\text{nombre de plaquettes dans EDTA-Formol}}{\text{nombre de plaquettes dans EDTA}} \times 100 = \text{pourcentage de plaquettes non agrégées}$$

Le produit à tester est d'autant plus inhibiteur de l'agrégation plaquettaire que le rapport se rapproche de 100.

On a ainsi déterminé que dans le lot (5 rats) traité par le composé de l'invention, le pourcentage obtenu est de 44 ± 13 alors que pour le lot témoin (5 rats), le pourcentage est de 6 ± 2.

b) mesure de l'agrégation plaquettaire au collagène

1,5 ml de sang citraté est additionné de 0,10 ml d'une solution contenant 10 µg de collagène par ml. Le milieu étant maintenu en agitation, le comptage des plaquettes est effectué sans interruption.

La diminution du nombre de plaquettes libres en fonction du temps est suivie en continu et permet de tracer une courbe dont la pente donne la vitesse initiale d'agrégation.

Pour le lot traité (5 rats) par le composé de l'invention, la vitesse initiale d'agrégation est de 2,33 ± 1,18, alors que pour le lot témoin non traité (5 rats), elle est de 12,44 ± 2,65.

L'étude de l'activité inhibitrice de l'agrégation plaquettaire a porté aussi sur l'action du composé de l'invention vis-à-vis du temps de saignement.

La méthode utilisée est une adaptation de la technique de L. STELLA, M.B., DONATI et G. de GAETANO, Thromb. Res., 1975, 7, 709-716.

L'expérimentation est effectuée sur le rat qui a reçu 65 heures, 41 heures et 17 heures auparavant un traitement per os de 200 mg du composé de l'invention en suspension dans 10 ml/kg d'une solution aqueuse de gomme arabique à 5 %. Après anesthésie au pentobarbital, la queue du rat est sectionnée à 5 mm de l'extrémité. Le sang est soigneusement épongé toutes les 15 secondes en prenant soin de ne pas toucher la plaie.

L'hémostase est atteinte lorsqu'il y a arrêt du saignement pendant une minute.

Les temps de saignement sont exprimés en secondes et les temps supérieurs à 1 200 (20 minutes) ne sont plus comptés.

On a ainsi déterminé que pour les témoins qui n'ont reçu que la gomme arabique, le temps moyen de saignement est de 390 secondes, alors que pour les animaux traités, il est supérieur à 1 200 secondes.

2°) Activité anti-thrombotique

Cette activité a été étudiée selon 2 méthodes :

a) méthode de la thrombose expérimentale sur fil de soie

Le principe de cette étude est une adaptation de la méthode de la thrombose expérimentale par circulation extra-corporelle, décrite par TERUHIKO UMETSU et KAZUKO SANAI (THROMB. HAEMOST., *39*, 1, 1978).

Sur le rat anesthésié par une injection intra-péritonéale de pentobarbital, la jugulaire gauche et la carotide externe droite sont mises à nu.

Le shunt artério-veineux est constitué par un cathéter central et deux cathéters latéraux ; un fil de soie naturelle blanche est introduit dans la partie centrale et la circulation rétablie pendant 20 minutes. Après arrêt de la circulation par clampage, le fil est retiré doucement et pesé immédiatement. Le poids moyen d'un fil de soie humide a été déterminé au préalable.

Le traitement est pratiqué 48 heures, 24 heures et 2 heures avant le début de la circulation sanguine dans le shunt par l'administration orale de 200 mg/kg du composé de l'invention en suspension dans 10 ml/kg de gomme arabique à 5 %, le témoin ne recevant que la solution de gomme arabique à 5 %.

On a ainsi déterminé que le poids moyen du thrombus dans les 2 lots de 10 rats était de 38,62 ± 1,18 mg pour les animaux témoins et de 25,00 ± 6,42 mg pour les animaux traités (—35 p. 100).

b) méthode de la thrombose veineuse à la vrille

Elle consiste en une adaptation de la méthode de FRIEDMAN et COLL (AM. J. PHYSIOL., 1960, *199*, 770-774). Une spirale métallique (bourre-pâte de dentiste) recoupée est insérée dans la veine cave inférieure du rat qui a reçu 48 heures, 24 heures et 2 heures auparavant un traitement per os de 200 mg/kg du composé à tester, en suspension dans 10 ml/kg d'une solution aqueuse de gomme arabique à 5 %.

Cinq heures après, cette spirale est prélevée avec le thrombus qu'elle retient, séchée délicatement par tamponnements successifs sur papier filtre et pesée. La spirale est ensuite débarrassée du thrombus, séchée de nouveau et pesée de nouveau. On obtient ainsi par différence le poids moyen du thrombus, qui est de 3,4 ± 0,4 mg pour les rats témoins (10 rats) qui n'ont reçu que la gomme arabique et 2,1 ± 0,3 mg pour les rats traités (10 rats) par le médicament de l'invention (—36 %).

Les études toxicologique et pharmacologique qui viennent d'être rapportées ont mis en évidence la faible toxicité du composé de l'invention et son excellente tolérance ainsi que ses intéressantes propriétés inhibitrices de l'agrégation plaquettaire et anti-thrombotique qui les rendent très utiles en thérapeutique humaine et vétérinaire.

Le médicament de l'invention peut être présenté pour l'administration orale sous forme de comprimés, comprimés dragéifiés, capsules, gouttes, granulés ou sirop. Il peut aussi être présenté pour l'administration rectale, sous forme de suppositoires et pour l'administration parentérale, sous forme de soluté injectable.

Chaque dose unitaire contient, avantageusement de 0,010 g à 0,500 g du composé de l'invention, les doses administrables journellement pouvant varier de 0,025 g à 1,50 g de principe actif en fonction de l'âge du malade et de la sévérité de l'affection traitée.

On donnera ci-après, à titre d'exemples non limitatifs, quelques formulations pharmaceutiques du médicament de l'invention.

1) Comprimés

Principe actif 0,150 g
Excipients : lactose, amidon, gomme arabique, stéarate de magnésium.

2) Comprimés dragéifiés

Principe actif 0,100 g
Excipients : polyvidone-excipient, gélatine, acide stéarique, amidon de maïs, lactose, sucre blanc, gomme arabique, tartrazine, coccine nouvelle.

3) Gélules

Principe actif 0,250 g
Excipients : amidon de maïs, stéarate de magnésium, sulfate de calcium anhydre.

4) Soluté injectable

Principe actif 0,100 g
Solvant isotonique q.s.p. 5 ml

5) Suppositoires

Principe actif 0,150 g
Excipient : triglycérides semi-synthétiques

Par ses propriétés inhibitrices de l'agrégation plaquettaire et anti-thrombotique, le médicament de l'invention est indiqué dans la prévention et le traitement des maladies provoquant une modification pathologique de l'agrégation plaquettaire, telle que les maladies thrombo-emboliques.

**Revendications** (pour les Etats contractants : BE, CH, DE, FR, GB, IT, LI, LU, NL, SE)

1. Dérivé de la tétrahydro-5,6,7,7a 4H-thiéno (3,2-c) pyridinone-2 répondant à la formule I

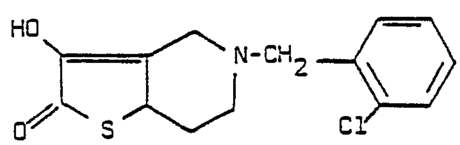

(I)

ainsi que ses sels minéraux ou organiques pharmaceutiquement acceptables.

2. Procédé de préparation du composé de formule I selon la revendication 1 caractérisé en ce que :

a) on soumet à une nitrosation le composé de formule II

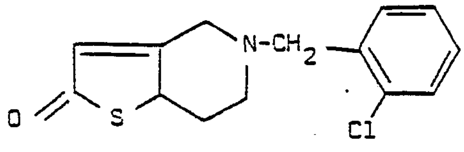

(II)

b) on hydrogène le céto-oxime obtenu de formule III

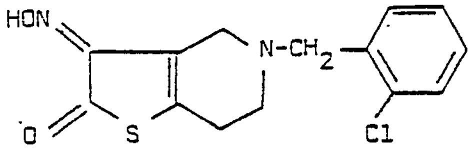

(III)

c) on fait subir un traitement acide au dérivé hydrogéné obtenu, de formule IV

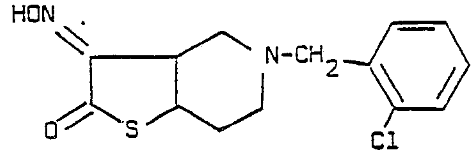

(IV)

pour obtenir le composé de formule I.

3. Procédé selon la revendication 2 caractérisé en ce que la nitrosation est effectuée par le nitrite de sodium en milieu acétique glacial et sous atmosphère inerte.

4. Procédé selon la revendication 3 caractérisé en ce que l'hydrogénation s'effectue en présence d'un catalyseur et d'acide perchlorique.

5. Procédé selon la revendication 3 caractérisé en ce que le traitement acide est effectué par de l'acide chlorhydrique à la concentration 2N, à reflux et sous atmosphère inerte.

6. Médicament caractérisé en ce qu'il contient, à titre de principe actif, le composé selon la revendication 1.

7. Médicament ayant des activités anti-agrégante plaquettaire et anti-thrombotique caractérisé en ce qu'il contient, à titre de principe actif, le composé selon la revendication 1.

8. Médicament selon les revendications 6 et 7 caractérisé en ce qu'il est présenté sous une forme appropriée à l'administration orale, parentérale ou rectale.

9. Médicament selon l'une des revendications 6, 7 ou 8 caractérisé en ce qu'il est présenté sous forme de doses unitaires contenant de 0,010 g à 0,500 g de principe actif.

**Revendications** (pour l'Etat contractant AT)

1. Procédé de préparation d'un dérivé de la tétrahydro-5,6,7,7a 4H-thiéno (3,2-c) pyridinone-2 répondant à la formule I

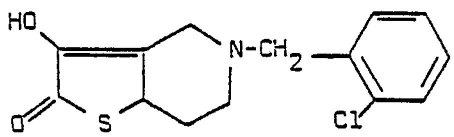

(I)

et ses sels minéraux ou organiques pharmaceutiquement acceptables, caractérisé en ce que :

a) on soumet à une nitrosation le composé de formule II

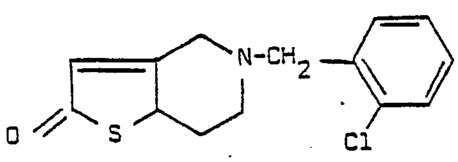

(II)

b) on hydrogène le ceto-oxime obtenu de formule III

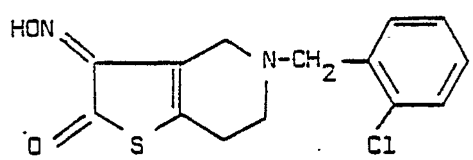

(III)

c) on fait subir un traitement acide au dérivé hydrogéné obtenu de formule IV

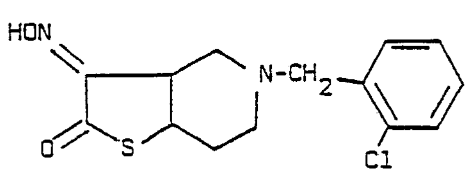

(IV)

pour obtenir le composé de formule I.

2. Procédé selon la revendication 1 caractérisé en ce que la nitrosation est effectuée par le nitrite de sodium en milieu acétique glacial et sous atmosphère inerte.

3. Procédé selon la revendication 2 caractérisé en ce que l'hydrogénation s'effectue en présence d'un catalyseur et d'acide perchlorique.

4. Procédé selon la revendication 2 caractérisé en ce que le traitement acide est effectué de préférence par de l'acide chlorhydrique à la concentration 2N, à reflux et sous atmosphère inerte.

5. Procédé de préparation d'un médicament, caractérisé en ce que l'on met sous une forme appropriée un composé obtenu selon l'une quelconque des revendications 1 à 4.

**Claims** (for the Contracting States : BE, CH, DE, FR, GB, IT, LI, LU, NL, SE)

1. 5,6,7,7a-Tetrahydro-4H-thieno (3,2-c) pyridin-2-one derivative, having formula I

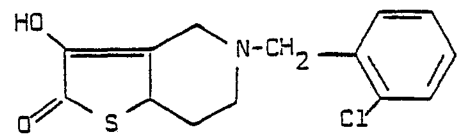

(I)

and its pharmaceutically acceptable inorganic or organic acid addition salts.

2. Process for the preparation of the compound of formula I according to claim 1, comprising :

a) nitrosating a compound of formula II

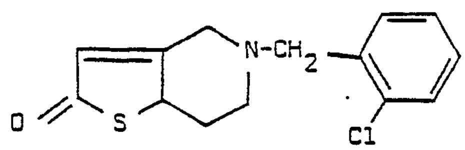

(II)

b) hydrogenating the resulting keto-oxime, of formula III

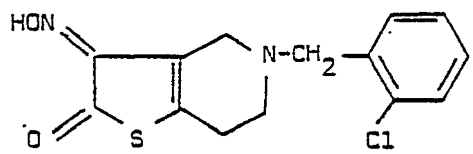

(III)

c) submitting the resulting hydrogenated derivative, of formula IV

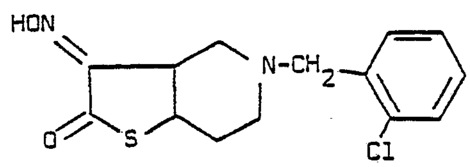

(IV)

to an acid treatment, to obtain the compound of formula I.

3. Process according to claim 2, characterized in that the nitrosation is effected with sodium nitrite in glacial acetic acid medium and under an inert atmosphere.

4. Process according to claim 3, characterized in that the hydrogenation is effected in the presence of a catalyst and of perchloric acid.

5. Process according to claim 3, characterized in that the acid treatment is effected with hydrochloric acid at 2N concentration, under refluxing conditions and under an inert atmosphere.

6. Medicament characterized in that it comprises, as active ingredient, the compound according to claim 1.

7. Medicament having anti-blood-platelet aggregation and anti-thrombosis activities, characterized in that it contains, as active ingredient, the compound according to claim 1.

8. Medicament according to claims 6 or 7, characterized in that it is formulated in a form suitable for oral, parenteral or rectal administration.

9. Medicament according to any one of claims 6, 7 or 8, characterized in that it is in dosage unit form, each unit dose containing from 0.010 g to 0.500 g active ingredient.

**Claims** (for the Contracting State AT)

1. Process for the preparation of 5,6,7,7a-Tetrahydro-4H-thieno (3,2-c) pyridin-2-one derivative, having formula I

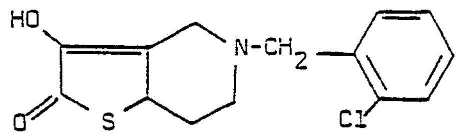 (I)

and its pharmaceutically acceptable inorganic or organic acid addition salts, comprising :

a) nitrosating a compound of formula II

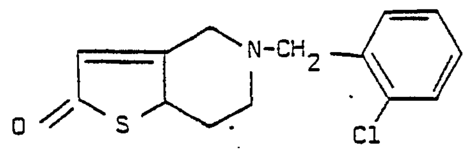 (II)

b) hydrogenating the resulting keto-oxime, of formula III

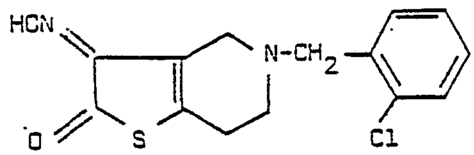 (III)

c) submitting the resulting hydrogenated derivative, of formula IV

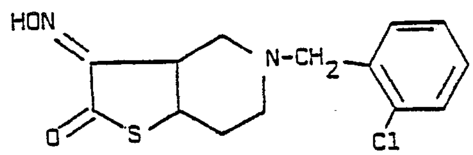 (IV)

to an acid treatment, to obtain the compound of formula I.

2. Process according to claim 1, characterized in that the nitrosation is effected with sodium nitrite in glacial acetic acid medium and under an inert atmosphere.

3. Process according to claim 2, characterized in that the hydrogenation is effected in the presence of a catalyst and of perchloric acid.

4. Process according to claim 2, characterized in that the acid treatment is effected with hydrochloric acid at 2N concentration, under refluxing conditions and under an inert atmosphere.

5. Process for the preparation of a medicament, characterized in that a compound obtained according to any one of claims 1 to 4 is formulated in a suitable form.

**Patentansprüche** (für die Vertragsstaaten : BE, CH, DE, FR, GB, IT, LI, LU, NL, SE)

1. Derivat von 5,6,7,7a-Tetrahydro (4H) thieno [3,2-c]-2-pyridinon entsprechend der Formel I

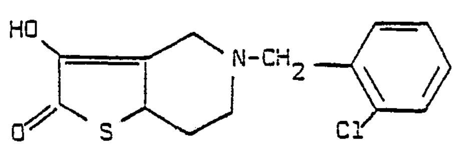 (I)

sowie seine anorganischen oder organischen, pharmazeutisch unbedenklichen Salze.

2. Verfahren zur Herstellung der Verbindung der Formel I nach Anspruch 1, dadurch gekennzeichnet, daß man

a) die Verbindung der Formel II

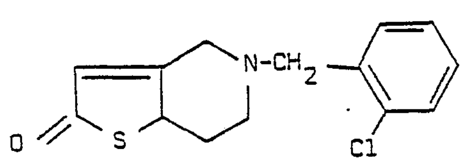 (II)

einer Nitrosierung unterwirft,

b) das erhaltene Ketooxim der Formel III hydriert

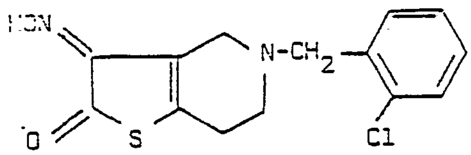

(III)

c) das erhaltene hydrierte Derivat der Formel IV

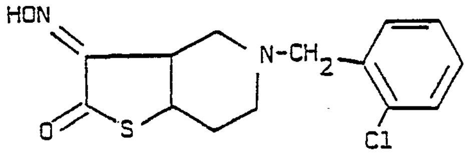

(IV)

einer Säurebehandlung unterwirft, um die Verbindung der Formel I zu erhalten.

3. Verfahren nach Anspruch 2, dadurch gekennzeichnet, daß die Nitrosierung mit Natriumnitrit in Eisessig als Medium und in einer inerten Atmosphäre durchgeführt wird.

4. Verfahren nach Anspruch 3, dadurch gekennzeichnet, daß die Hydrierung in Gegenwart eines Katalysators und von Perchlorsäure durchgeführt wird.

5. Verfahren nach Anspruch 3, dadurch gekennzeichnet, daß die Säurebehandlung mit Salzsäure mit einer Konzentration von 2N unter Rückfluß und unter einer inerten Atmosphäre durchgeführt wird.

6. Medikament, dadurch gekennzeichnet, daß es als Wirkstoff die Verbindung nach Anspruch 1 enthält.

7. Medikament mit die Zusammenballung der Blutplättchen verhindernden Eigenschaften und antithrombotischen Eigenschaften, dadurch gekennzeichnet, daß es als Wirkstoff die Verbindung nach Anspruch 1 enthält.

8. Medikament nach Anspruch 6 und 7, dadurch gekennzeichnet, daß es in einer für die orale, parenterale oder rektale Verabreichung geeigneten Form vorliegt.

9. Medikament nach einem der Ansprüche 6, 7 oder 8, dadurch gekennzeichnet, daß es in Form von Einheitsdosen vorliegt, die 0,010 bis 0,500 g Wirkstoff enthalten.

**Patentansprüche** (für den Vertragsstaat AT)

1. Verfahren zur Herstellung eines Derivats von 5,6,7,7a-Tetrahydro-(4H) thieno [3,2-c]-2-pyridinon entsprechend der Formel I

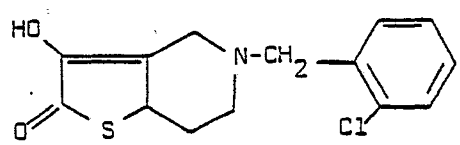

(I)

und seiner pharmazeutisch unbedenklichen anorganischen oder organischen Salze, dadurch gekennzeichnet, daß man

a) die Verbindung der Formel II

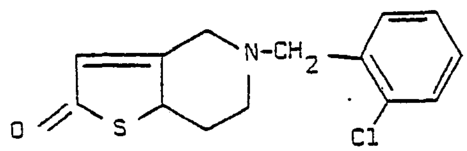

(II)

einer Nitrosierung unterwirft,

b) das erhaltene Ketooxim der Formel III hydriert

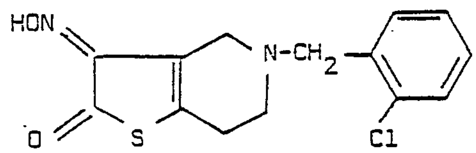

(III)

c) das erhaltene hydrierte Derivat der Formel IV

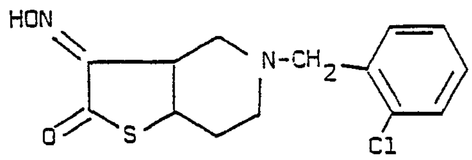

(IV)

einer Säurebehandlung unterwirft, um die Verbindung der Formel I zu erhalten.

2. Verfahren nach Anspruch 1, dadurch gekennzeichnet, daß die Nitrosierung mit Natriumnitrit in Eisessig als Medium und in einer inerten Atmosphäre durchgeführt wird.

3. Verfahren nach Anspruch 2, dadurch gekennzeichnet, daß die Hydrierung in Gegenwart eines Katalysators und von Perchlorsäure durchgeführt wird.

4. Verfahren nach Anspruch 2, dadurch gekennzeichnet, daß die Säurebehandlung vorzugsweise mit Salzsäure einer Konzentration von 2N unter Rückfluß und in inerter Atmosphäre durchgeführt wird.

5. Verfahren zur Herstellung eines Medikaments, dadurch gekennzeichnet, daß man eine nach irgendeinem der Ansprüche 1 bis 4 erhaltene Verbindung in eine geeignete Form bringt.